# EUROPEAN PATENT SPECIFICATION

(11) **EP 1 322 293 B1**
(45) Date of publication and mention of the grant of the patent: **27.09.2006**
(21) Application number: 01963156.3
(22) Date of filing: 30.08.2001
(51) Int. Cl.: A61K 9/20

(54) **MODIFIED RELEASE FORMULATION**
ARZNEIMITTEL MIT VERZÖGERTER WIRKSTOFFABGABE
FORMULATION A LIBERATION MODIFIEE

(30) Priority: 05.09.2000 SE 0003125
(43) Date of publication of application: 02.07.2003
(73) Proprietor: AstraZeneca AB, 151 85 Södertälje (SE)
(72) Inventor: EKLUND, Marianne, S-433 70 Savedalen (SE); LOFROTH, Jan-Erik, S-431 83 Molndal (SE); SKANTZE, Urban, S-431 83 Molndal (SE)
(74) Representative: Nelson, Michael Andrew
(86) International application number: PCT/GB2001/003861
(87) International publication number: WO 2002/019990

(56) References cited:
- WO-A-94/15643
- WO-A-96/16638
- WO-A-96/16639
- WO-A-96/40163

## Description

### TECHNICAL FIELD

The present invention relates to pharmaceutical modified release formulations containing a modified water-soluble polysaccharide, in particular to formulations containing a modified water-soluble polysaccharide with improved compaction properties, more particularly to modified release formulations containing a modified water-soluble polysaccharide as the sole or major excipient in the formulation.

### BACKGROUND ART

In many pharmaceutical applications a tablet is the preferred dosage form for a drug. A tablet formulation will generally meet the requirements of safety, simplicy and patient compliance. An important parameter in a tablet formulation is the compaction properties of the substances of the formulation, Sheth et al in Pharmaceutical Dosage Forms: Tablets. Volume 1 (Eds Liebermann and Lachman; Marcel Dekker, NY 1980; p109). Good compaction properties (as indicated by, for example, the hardness of the tablet) can be obtained by the use of binders and fillers. However, the amount of such agents that can be incorporated is limited, as too much of the filler and/or binder will give tablets that are too large. Also, the function of a tablet, for example the release profile or dissolution characteristics, are influenced by the type of substances present in the tablet.

In recent years there has been a large increase in the development and use of so called modified release (MR) formulations. Such formulations are designed to release a drug more or less slowly after ingestion compared to administering the drug per se or via an inert formulation. MR formulations can give improved therapeutic effects, reduced incidence of adverse effects and simplified dosing regimens. Generally MR tablets release the drug contained therein over several hours, typically more than 3 hours. Generally the drug will be released from the MR formulation in less than 30 hours. Several different types of modified release formulations are known, for example, Langer and Wise (Eds) "Medical applications of Controlled release", vols I and II, CRC Press Inc, Boca Raton, 1984; Robinson and Lee (Eds) "Controlled drug delivery - fundamentals and applications", Marcel Dekker, NY, 1957; Bogentoft and Sjögren, in "Towards better safety of drugs and pharmaceutical products" (Ed: Braimer), Elsevier, 1980; Sandberg "Extended-release metoprolol", Thesis, Uppsala University, 1994. These known modified release formulations use a variety of mechanisms to control the release of an active substance from the formulation. The particular mechanism selected being largely dependent upon the therapeutic aims of the formulation. Examples of mechanisms exploited in modified release formulations include the control of dissolution, diffusion, swelling, osmotic pressure, complexation, ion-exchange or erosion of the tablet.

Polymers can be used to form a matrix in which an active substance is dispersed, the properties of the polymer are then utilised control the rate at which the active is released from the formulation. Such polymers include polysaccharides, especially the cellulose derivatives hydroxypropyl methylcellulose (HPMC) and hydroxypropyl cellulose (HPC), see "Handbook of Pharmaceutical Excipients" (Ed: A Wade and P J Weller, Pharmaceutical Press, London 1994). The properties of the polymer when placed in water, especially in a physiological environment can be used to control the rate of release of an active substance from the MR formulation. For example, polymers such as HPMC often develop a gel-like outer layer that dissolves or erodes, thereby controlling the rate of water ingress into the MR formulation. The release of active substance from such MR formulations is believed to be controlled by a combination of several factors, including the properties of the active substance, the diffusion properties of the active substance or a solution thereof once dissolved, the penetration rate of the water into the polymer matrix, the swelling of the polymer matrix, and the rate of dissolution of the outer polymer gel layer.

However, many polymers used in MR formulations are sensitive to external factors and as a result are not sufficiently robust to be used in commercial MR formulations. Of particular importance is the sensitivity of the polymers to salt and surface active agents, e g bile salts and lipids. For example, ionic polymers are inherently instable due to ionic interactions which decrease the swelling and hydration of the polymer once the formulation is in contact with, for example, the gastro-intestinal content. Non-ionic polymers, e g HPMC and HPC, also show considerable interaction with ionic substances and with surface active agents, see, e g, G Karlström et al (e g J Phys Chem **89**, 4962 (1985); *ibid* **94**, 5005 (1990); In *Organized Solutions* (Ed S Friberg and B Lindman) Marcel Dekker, NY 1992, p 49), J-E Löfroth et al (Progr Colloid Polym Sci **84**, 73 (1991); *ibid* **84**, 78 (1991)). The presence of ionic substances and/or surface active agents lowers the cloud-point of these polymers, and can result in a collapse of the matrix. Moreover, such interactions can occur between the polymers and excipients present in the formulation, e g fillers, binders, etc. There is therefore a need for alternative polymer materials for the use in pharmaceutical formulations, especially modified release formulations which are less sensitive to such external factors.

The cellulose derivative hydroxyethyl cellulose (HEC) is a water-soluble, non-ionic polymer which is approved for the use in pharmaceutical formulations, see "Handbook of Pharmaceutical Excipients" (Ed: A Wade and P J Weller, Pharmaceutical Press, London 1994). HEC exhibits some sensitivity to external factors like salt, surface active agents, temperature, etc, but not to the extent as other non-ionic cellulose derivatives like HPMC, HPC, methyl cellulose etc, or ionic polymers, see e g C D Melia, Crit Rev Ther Drug Carrier Systems **8,** 395 (1991); Product Information from Hercules Ltd on Aqualon products: Natrosol® ; and Information from Union Carbide Corporation : Cellosize® Hydroxyethyl Cellulose. Also, HEC is available with many different molecular weights, which may facilitate the manufacture of MR formulations comprising a matrix of polymer with different erosion and release characteristics without the need for substantial amounts of other materials that could interfere with the release properties of the HEC.

To take full advantage of the desirable properties of HEC, for example its swelling and dissolution characteristics, robustness in many different environments, availability of different molecular weights etc, it is desirable that the formulation contains typically more than 30-40 % w/w of the rate controlling polymer. However, HEC has poor compaction properties, and gives formulations (such as tablets or granules) that are soft and friable compared to tablets formed from other polymers such as HPMC. In view of the poor compaction properties of HEC it has mainly been used only as a minor component in formulations, for example as a binder or a film-coating agent for tablets. In such formulations the rate of release of drug from the tablet is generally not significantly affected by the properties of the HEC.

However, polymer blends are prone to phase separation when solubilized, Piculell and Lindman, Adv Colloid Interface Sci **41**, 149 (1992). Therefore, it is desirable to avoid ingredients other than the rate controlling excipient and the drug and thereby minimise these effects and provide a reproducible and safe rate of drug release. Also, such formulations would allow lower cost manufacturing compared to formulations requiring more than one rate controlling excipient.

Other polymers which are not significantly sensitive to external parameters, such as ionic strength are certain natural, water soluble polymers, for example locust bean gum (LBG), tragacanth gum or pectin. These polymers have not been used *per se* as rate controlling polymers in MR-formulations, again most likely due to inherent properties which render the polymers unsuitable for compaction unless used in combination with other materials in substantial amounts, which then would give formulations that do not utilize in an optimal way the possibilities such polymers can provide for in the control of the release of an active substance.

WO94/15643 discloses compositions comprising konjac galactomannan hydrocolloid. WO96/16638 discloses compositions comprising hydrocolloid gum and another excipient that aids in sustained release and WO96/16639 discloses compositions comprising hydrocolloid gum and a dispersing enhancing amount of another excipient. WO96/40163 discloses compositions comprising purified galactomannan hydrocolloid.

### PRIOR ART

HEC has been used in drug formulations, but generally only in combination with other materials to provide a compressible mixture. Generally the formulations comprise low amounts of HEC. For example aliphatic alcohols and HEC are used together in formulations described in US *3,965,256.* Drug delivery formulations containing less than 20% by weight HEC as a binder are described in US 4,851,233. Mixtures of HEC and other polymers are reported in J Kor Pharm Sci **17**,47 (1987), US 5,004,613 Drug Dev Ind Pharmacy **20**, 2645 (1994) and US 5,451,409.

Formulations based on HEC either in larger amounts or as the only excipient as described in Gulde and Voigt (Pharmazie **38**, 542 (1983); Aqualon Company (Research Disclosure **288**, 233 (1988) and Matsuo et al [Chem Phann Bull **43**,311 (1995); Int J Pharm **138**, 225 (1996].

Gulde and Voigt disclose formulations in which ingredients (drug, HEC, talc, amylum) are mixed and compressed. The major amount of the tablet was amylum (starch), which amounted to about 56 % w/w.

The formulations disclosed in the above Aqualon Company reference have tablet contents of HEC of 35 % w/w and also other ingredients. Thus, e.g. the contents of di-calcium phosphate was between 32-40 % w/w.

The formulations (multi-layer tablets) disclosed in the two Matsuo references were prepared by complicated process in which first a drug core was compressed, then a HEC disk is compressed with half the amount HEC needed for the tablet. The drug core is then placed on this disk and a die filled with HEC and the system compressed to give a core-shell tablet.

However, the product quality, in terms of tablet hardness, was not assured in any of these references. Tablet breaking force, e g in the 1.4 kP region (13.4 N as reported by Gulde and Voigt) is not acceptable for large scale production and handling of tablets. When commercially available HEC is used to prepare tablets by compression, the tablets exhibit an unacceptably low breaking force unless substantial amounts of other excipients are used such as binders or fillers etc. In these known formulations it is not possible to fully utilize the advantageous release rate controlling properties of the HEC polymer. We have found that the same problem is present with other polysaccharides such as LBG, which cannot be sufficiently compacted when it is used as the sole or major (i.e. >20% by weight) excipient of a tablet. Attempts to obtain a robust MR formulation wherein the polymer matrix (eg as a gelling matrix) is HEC or another polysaccharide with unsuitable binding or other surface properties for compaction have until now not been successful, MR formulations containing as a matrix such polysaccharides cannot be handled under normal commercial conditions without breaking or falling apart because such formulations do not have acceptable compressibility properties.

We have surprisingly found that by modifying polysaccharides which hitherto have been considered unsuitable for compaction, it is possible to provide modified release formulations using such polysaccharides (such as HEC) having good compaction properties and a high tablet hardness. Many of the modified polysaccharides are not sensitive to surface active agents (for example bile salts, lipids) or increased ionic strengths due to the presence of salts, etc, present either as part of the formulation or in an external dissolution medium (for example the gastro-intestinal contents).

### DISCLOSURE OF THE INVENTION

According to a first aspect of the present invention there is provided a pharmaceutical modified release formulation comprising a pharmacologically active substance and a modified water-soluble polysaccharide, which modified water-soluble polysaccharide is obtainable by:
a) forming a precipitate of a water-soluble polysaccharide by contacting a solution of said polysaccharide with a solvent in which said polysaccharide is poorly soluble or insoluble; or
b) milling a water-soluble polysaccharide,
wherein the water-soluble polysaccharide is a hydroxyethyl cellulose.

### Polysaccharide

A suitable water-soluble polysaccharide for modification by means of the processes described in (a) or (b) above is one with properties that typically render it unsuitable for use in normal compaction, for example by means of a Killian excentric column press or a Diaf press. Polysaccharides that are unsuitable for use in normal compaction are those which when compressed to form a tablet have a tablet hardness of 6kP or less, preferably 5.5kP or less more preferably less than 5kP, for example in the range of from 0.1 to 4kP. Tablet hardness refers to the force required to crush a compressed tablet of the polysaccharide between two parallel plates or jaws. The tablet hardness may be measured by preparing a tablet by compressing 200mg of the polysaccharide with a tablet press (for example a Killian excentric column press SP200 or a Diaf tablet press) using a compression force of approximately 20kN with an 8mm die and standard convex punches. The hardness of the resulting tablet may then be assessed by measuring the force required to crush the tablet, for example using a Schleuniger-4M Tablet Hardness Tester.

Preferably the water-soluble polysaccharide has a solubility in water at 25°C, pH 7 of at least 0.01% by weight, more preferable at least 0.1% by weight.

Water-soluble polysaccharides suitable for modification include, for example HEC (Hydroxy Ethyl Cellulose). It is especially preferred that the water-soluble polysaccharide is HEC.

Water-soluble polysaccharides are commercially available or may be prepared using known techniques. For example, HEC is a hydroxyethyl ether of cellulose prepared by etherifying one or more of the reactive hydroxyl groups in cellulose with ethylene oxide. Typically etherification is carried out by treating cellulose with a base, such as sodium hydroxide followed by reaction with ethylene oxide. Depending upon the reaction conditions, the etherification results in the introduction of hydroxyethoxy groups or short hydroxy[ethoxy]ₙ polymeric side chains attached at one or more of the positions previously occupied by a hydroxyl group in the cellulose molecule, wherein n is typically 2 to 6. Generally there are three hydroxyl groups available for etherification in each anhydroglucose unit of a cellulose molecule. The number of hydroxyls etherified in each anhydroglucose unit (the degree of substitution) affects the physical properties of the resulting HEC. Generally, the HEC will have a degree of substitution of from 0.5 to 3, preferably from 1 to 2.5.

HEC is available in a wide range of molecular weights. Generally the HEC will have a weight average molecular weight in the range of from 80k Daltons to 1500k Daltons, for example from 250kDaltons to 1200kDaltons as measured using size exclusion chromatography (SEC) against suitable calibration standards.

HEC is available commercially, for example as Natrosol^{™} from Hercules Ltd.

### Modification by Precipitation

When the water-soluble polysaccharide is modified by precipitation ((a) above) a solution of the polysaccharide is contacted (for example by mixing) with a solvent in which the polysaccharide is poorly soluble or insoluble. Thus the solvent is a poor solvent for the polysaccharide and therefore results in the formation of a precipitate of the polysaccharide. By "poor solvent" is meant in this specification the accepted definition by a poor solvent as based on the concepts of the theta temperature, and the second virial coefficient (E G Richards *An introduction to physical properties of large molecules in solution,* IUPAB Biophysics Series, Cambridge Press, Cambridge 1980). Therefore, at temperatures lower than the theta temperature a solvent becomes a poor solvent. The theta temperature is defined by the χ interaction parameter of the energy of mixing.

A solution of the polysaccharide is prepared by dissolving the polysaccharide in a first solvent. The first solvent is one in which the water-soluble polysaccharide has a solubility at 25°C of at least 0.01% by weight, preferably at least 0.1 % by weight. Preferably the solution has a high concentration of the polysaccharide to give efficient precipitation of the modified polysaccharide. The upper limit of polysaccharide concentration in the solution will generally be limited by the viscosity of the solution, which increases as the polysaccharide concentration increases. Preferably the solution contains at least 1% w/w of the water-soluble polysaccharide.

As will be realised, the selection of the first solvent will depend upon the nature of the polysaccharide. A suitable first solvent includes, but is not limited to, water, formic acid (preferably a 70% solution in water), dimethyl sulfoxide, methyl formamide or a mixture thereof, for example a water/ethanol mixture in which the polysaccharide is soluble (for example a mixture comprising 70 parts by weight water and 30 parts by weight ethanol). When the water-soluble polysaccharide is HEC the first solvent preferably comprises water or a mixture of water and ethanol containing less than 40%, more preferably less than 30% and especially less than 20% by weight of ethanol.

The modified water-soluble polysaccharide is precipitated from the solution by contacting the solution with a poor solvent for the polysaccharide. The poor solvent may be added to the solution containing the polysaccharide. Alternatively, the solution containing the polysaccharide may be added to the poor solvent.

Suitably, the poor solvent is one in which the polysaccharide has a solubility at 25°C of less than 0.1% by weight, preferably less than 0.01% by weight.

As will be realised, selection of the poor solvent is dependent upon the properties of the polysaccharide. The poor solvent should also be selected such that the first solvent has a sufficient solubility therein to allow the polysaccharide to be precipitated from the solution. The poor solvent is preferably an organic solvent in which the polysaccharide is poorly soluble. A suitable organic solvent includes, but is not limited to a ketone (for example acetone or methyl ethyl ketone) an alcohol (for example ethanol, methanol, n-propanol or isopropyl alcohol), an ester (for example ethyl acetate, ethyl lactate or methyl salicylate), an organic acid (for example glacial acetic acid), an ether (for example tetrahydrofuran), a nitrile (for example acetonitrile), an aromatic hydrocarbon (for example toluene) or an aliphatic hydrocarbon (for example hexane), or mixtures thereof.

In a preferred embodiment the first solvent is miscible in all proportions with the poor solvent. This gives rapid precipitation of the polysaccharide from the solution. In this embodiment, the poor solvent is a water-miscible organic solvent in which the polysaccharide is poorly soluble or insoluble. Suitable water-miscible organic solvents include a water-miscible alcohol, for example methanol, ethanol, n-propanol, or isopropyl alcohol; a water-miscible ether, for example tetrahydrofuran; a water-miscible nitrile, for example acetonitrile; or a water-miscible ketone, for example acetone or methyl ethyl ketone. It is especially preferred that the water-miscible organic solvent is a water-miscible alcohol (especially methanol, ethanol or isopropyl alcohol) or a water-miscible ketone (especially acetone).

When the poor solvent is a water-miscible organic solvent, the organic solvent may be used as a solution containing a small amount of water, preferably less than 30%, more preferably less than 20% by weight water. For example, a solution comprising 20 parts by weight water and 80 parts by weight of a water-miscible organic solvent (e.g. acetone or ethanol) may be used. This may provide additional control over the rate of precipitation and/or the physical form of the precipitated modified polysaccharide.

The addition of the poor solvent to the polysaccharide solution (or vice versa) is preferably performed under agitation to ensure rapid mixing and efficient precipitation of the modified polysaccharide from the solution. Agitation may be provided using conventional techniques well known in the art, for example by stirring or shaking.

Following precipitation the modified water-soluble polysaccharide may be isolated using conventional techniques such as filtration, centrifuging or lyophilisation.

Preferably the isolated modified polysaccharide is washed with further of the poor solvent. If required the modified polysaccharide may be dried for example by air drying, oven drying, vacuum drying or freeze drying.

### Modification by Milling

When the modified water-soluble polysaccharide is prepared by milling ((b) above) a water-soluble polysaccharide is milled in a milling device that utilizes balls, knives or other methods for a defined time, preferably under controlled temperature conditions (for example with cooling). A suitable milling device includes, a ball mill, a knife mill, an air-jet mill or a hammer mill.

When a knife mill is used typical milling conditions suitable for modifying the polysaccharide include at least one knife blade rotating at 1-30000 rpm, preferably 10000-30000 rpm, more preferably approximately 20000 rpm. When a ball mill is used suitable conditions include the use of milling balls (preferably with a diameter of 2 cm).

The milling time required to give the modified water-soluble polysaccharide will depend to some extent upon the polysaccharide and the milling conditions. In the case of water-soluble polysaccharides such as HEC a milling time of from 0.25 to 600 minutes, more preferably from 1 to 30 minutes, still more preferably from 3 to 20 minutes and especially from 5 to 15 minutes in a knife mill or a ball mill is sufficient. The milling may be carried out continuously or, more preferably in intervals. For example if the total milling time is 15 minutes the polysaccharide may be milled continuously for 15 minutes. Alternatively, the polysaccharide may be milled in three separate five minute intervals. Milling in intervals, for example of from 30 seconds to 10 minutes (preferably from 2 minutes to 5 minutes) is preferred because localised heating of the polysaccharide during the milling is minimised, thereby reducing the possibility of degrading the polysaccharide. Preferably the milling is performed under controlled temperature conditions, for example by providing cooling to minimise heating of the polysaccharide. The cooling is preferably sufficient to maintain the polysaccharide at a temperature below its glass transition temperature. Cooling may be provided by, for example, a water-circulation system which pumps water through conduits in the body of the mill.

Milling of the polysaccharide is continued until the required degree of modification has been attained. The degree of modification is that required to provide a modified water-soluble polysaccharide with increased compressibility and thus increased tablet hardness compared to a tablet prepared using a un-modified polysaccharide.

The degree of modification of the polysaccharide produced by milling or by the precipitation (process (a) defined above) may be determined by measuring the tablet hardness of a tablet of the modified water-soluble polysaccharide. A tablet of the polysaccharide is prepared by compressing 200mg of the modified water-soluble polysaccharide with a tablet press (for example a Killian excentric column press SP200 or a Diaf tablet press) using a compression force of approximately 20kN with an 8mm die and standard convex punches. The hardness of the resulting tablet is then determined by measuring the force required to crush the tablet, for example using a Schleuniger-4M Tablet Hardness Tester. Preferably the modified polysaccharides provide a tablet hardness of more than 6kP, preferably at least 7kP, for example from 6.5kP to 40kP, more preferably from 7 to 35kP and especially from 8 to 35kP.

Alternatively, a skilled person will be able to ascertain whether the water-soluble polysaccharide is sufficiently modified following milling or precipitation by reference to suitable analytical methods, including FT-Raman spectroscopy, powder X-ray diffractometry and changes in the glass transition temperature (Tg) of the polysaccharide. Such techniques can be used to compare the properties of the polysaccharide before and after milling and thereby correlate such properties with those required to provide the desired compressibility and tablet hardness. Additional parameters that may be used to determine the degree of modification of the polysaccharide include, for example surface modifications, particle size and morphology changes. Such changes may be detected using a variety of techniques, for example SEM, powder X-ray diffractometry, FT-Raman spectroscopy, dynamic mechanical thermal analysis (DMTA) or calorimetry.

The modified water-soluble polysaccharides can be utilized to prepare modified release formulations with material which would otherwise be impossible to compress to acceptable hardness. Such formulations are useful to obtain improved drug administration.

### Pharmacologically Active Substance

Any type of active drug substance, for which a modified release profile is desirable, can be utilized and incorporated as part of the formulation by methods known for those skilled in the art. The pharmacologically active substance may be water soluble or water-insoluble. Examples of suitable pharmacologically active substances include, but are not limited to butyl paraben, fluvastatin, and pharmaceutically acceptable salts thereof, especially the sodium salt, glycine, N-[1-cyclohexyl-2-[2-[[[[4-[(hydroxyimino)aminomethyl]-phenyl]methyl]amino]carbonyl]-1-azetidinyl]-2-oxoethyl]-, ethyl ester, [S-(R*,S*)]-) (as described in Example 17 of WO 97/23499), metoprolol or a pharmaceutically acceptable salt thereof, especially the succinate and tartrate salts, or felodipine.

### Modified Release Formulation

The modified release formulation according to the present invention may be prepared by mixing the modified water-soluble polysaccharide with the pharmacologically active substance and compressing the resulting mixture.

Suitable methods for preparing the modified release formulations according to the present invention include those described below and further illustrated in the Examples.

A suitable method for preparing the modified release formulation is based on direct compression, wherein a mixture comprising the modified water-soluble polysaccharide and pharmacologically active substance is compressed. Compression may be provided by any suitable means, for example by a conventional tablet press.

Another method for preparing the modified release formulation comprises dry or wet granulation of the modified water-soluble polysaccharide to provide granules of the modified polysaccharide. The granules may then be mixed with the pharmacologically active substance and the resulting mixture compressed to give a modified release formulation according to the present invention.

In another embodiment the modified water-soluble polysaccharide is wet or dry granulated together with the pharmacologically active substance to provide a modified release formulation comprising granules containing a mixture of the modified polysaccharide and the phannacologically active substance. The resulting granules may then be compressed into tablets using, for example, a tablet press as described above. Alternatively, the granules may be filled into a capsule or sachet to provide a unit dosage form containing a sufficient quantity of granules necessary to provide the required dose of the pharmacologically active substance.

Apparatus and methods for wet and dry granulation are well known in the art, for example as described in Encyclopedia of Pharmaceutical Technology, Vol 7, Swarbrick and Boylan, published by Marcel Dekker Inc. pp 120-160.

Dry granulation may be performed by well known procedures, for example, by gently compressing the polysaccharide in a mortal and pestle to form granules. The resulting granules are then sieved to obtain granules of the desired particle size which are then mixed with the pharmacologically active substance and compressed into tablets. Alternatively, modified release granules may be obtained directly by dry granulating a mixture of the modified water-soluble polysaccharide and the pharmacologically active substance.

Wet granulation may be carried out by granulating the modified polysaccharide using a granulation liquid which acts to bind the modified polysaccharide into granules. Suitable granulation liquids include an organic solvent (especially a volatile organic solvent such as acetone or a volatile alcohol such as methanol or ethanol), a polymer solution (for example a PVP (poly vinylpyrrolidone), HPMC (hydroxypropylmethyl cellulose), HPC (hydroxypropyl cellulose) or HEC (hydroxyethyl cellulose) solution); a sugar solution, for example a sorbitol or manitol solution. When the granulation liquid comprises a polymer solution or sugar solution, the granulation liquid is based on a suitable solvent for the substance present in the granulation liquid to be used, e g water, ethanol, acetone, etc. Preferably the granules are dried prior to use, for example by vacuum drying or oven drying. Preferably drying is carried out at a temperature lower than the glass transition temperature of the modified water-soluble polysaccharide. If desired the granules may be sieved prior to compression into tablets using a tablet press.

In another embodiment of the invention the modified water-soluble polysaccharide is wet granulated as described above together with the pharmacologically active substance, thereby providing a modified release formulation in the form of granules comprising the modified water-soluble polysaccharide and pharmacologically active substance. In this embodiment the pharmacologically active substance may be mixed with the modified water-soluble polysaccharide and then granulated using the granulation liquid. Alternatively, the pharmacologically active substance may be dissolved or suspended in the granulation liquid used to granulate the modified water-soluble polysaccharide.

We have surprisingly found that when the modified water-soluble polysaccharide is a modified HEC it can be used in wet granulation processes to give granules with improved physical form compared to the use of an un-modified HEC. We have found that un-modified HEC in wet granulation tends to agglomerate uncontrollably and often results in the formation of a single mass of material which is impossible to granulate.

Preferably, the modified release formulation according to the present invention is a formulation adapted for oral administration, for example a tablet, a capsule or a sachet. It is especially preferred that the modified release formulation is a compressed tablet adapted for oral administration. Such dosage forms may be prepared using conventional techniques well known to those skilled in the art.

When the modified release formulation is a compressed tablet formulation it is preferred that the modified water-soluble polysaccharide and pharmacologically active material are compressed together shortly after preparing the modified water-soluble polysaccharide (preferably less than 1 month, more preferably less than 1 week, and especially less than two days after preparing the modified polysaccharide). We have surprisingly found that this provides a tablet that maintains its hardness for a prolonged period of time.

Preferably the modified water-soluble polysaccharide comprises at least 25%, more preferably at least 30% and still more preferably at least 40% and especially at least 50% by weight of the modified release formulation. The upper limit of the modified water-soluble polysaccharide content in the formulation will be determined by the other components in the formulation such as the pharmacologically active material. Thus when the MR formulation contains a high drug loading the formulation will necessarily contain lower amounts of the modified water-soluble polysaccharide. Preferably, however, the modified water-soluble polysaccharide is the major excipient present in the formulation. By major excipient is meant that excluding the pharmacologically active material, the modified water-soluble polysaccharide forms at least 50%, more preferably at least 60% and especially at least 70% by weight of the remaining formulation. Generally the modified release formulation will contain from 25 to 65% by weight, more preferably from 30 to 50% by weight of the modified polysaccharide.

In a preferred embodiment the modified water-soluble polysaccharide is the only polymeric excipient present in the modified release formulation, because this reduces the possibility of undesirable interactions with other polymeric excipients, whilst maximising the desirable, robust, release properties of the modified polysaccharide.

In view of the above preferences a preferred modified release formulation according to the present invention is a compressed modified release tablet adapted for oral administration comprising a pharmacologically active substance and a modified water-soluble polysaccharide, wherein the modified water-soluble polysaccharide is obtainable by:
a) forming a precipitate of a water-soluble polysaccharide by contacting an aqueous solution of said polysaccharide with a water-miscible organic solvent in which said polysaccharide is poorly soluble/insoluble; or
b) milling for a period of at least 5 minutes a water-soluble polysaccharide in a knife mill containing at least one blade rotating at 10000 to 30000 rpm,
wherein the water-soluble polysaccharide is a hydroxyethyl cellulose.

In this embodiment of the invention it is preferred that when the process (a) is used to obtain the modified HEC that the water miscible solvent is selected from acetone and ethanol. Optionally the water-miscible organic solvent may contain a small amount of water provided that the presence of the water does not prevent precipitation of the modified HEC. For example the water-miscible solvent may contain up to 30% w/w water, for example from 5 to 20% w/w water.

According to another embodiment of the present invention there is provided a modified release tablet formulation (preferably a compressed tablet formulation) comprising a pharmacologically active substance and a modified water-soluble polysaccharide, wherein the modified water-soluble polysaccharide is a modified hydroxyethyl cellulose, wherein the tablet has a hardness of more than 6kP. preferably at least 7kP, for example from 6.5 to 40kP, preferably from 7 to 35kP. The tablet hardness here refers to the force required to crush the tablet comprising the modified water-soluble polysaccharide and the pharmacologically active substance. The tablet hardness may be measured using a standard tablet hardness tester, such as the Schleuniger-4M Tablet Hardness tester hereinbefore described above in relation to the modified water-soluble polysaccharides.

The release of the pharmacologically active substance from the formulation is determined by, amongst other things, the rate at which the modified water-soluble polysaccharide hydrates (swells) and dissolves and the rate at which the active substance diffuses through the polysaccharide. The rate of release may be modified by, for example, by mixing different molecular weights of one modified polysaccharide, for example a mixture of different molecular weights of HEC. This is advantageous because it minimises the risk of incompatibilities, such as phase separation, which can arise through using a blend of different polysaccharides or polymers.

The modified release formulation optionally contains other additional components to provide further functional features to the formulation, provided that the presence of such additional components do not adversely affect the properties of the formulation, for example the tablet hardness, the release profile of active substance or induce phase separation as hereinbefore discussed. The additional components may be used, for example, to influence flow and other manufacturing parameters, to increase/decrease of solubility of the pharmacologically active substance; ionic strength; pH; release rate; etc. Optional additional components in the modified release formulation include for example, polymers; surfactants; lipids; salts; binders; fillers; lubricants; disintegrants; glidants; colour additives; flavours; preservatives; anti-oxidants and other excipients conventionally used in pharmaceutical formulations. Generally the amount of optional additional components is a small as possible to minimise the possibility of adversely interacting with the modified polysaccharide. Preferably, when present any optional additional components represent less than 10% by weight of the modified release formulation.

The surprisingly high compressibility of the modified water-soluble polysaccharide provides modified release formulations that can be handled under normal commercial conditions without breaking or crumbling.

According to another aspect of the present invention there is provided a pharmaceutical modified release formulation according to the first aspect of the present invention for use in therapy.

In the context of the present specification, the term "therapy" also includes "prophylaxis" unless there are specific indications to the contrary. The terms "therapeutic" and "therapeutically" should be construed accordingly.

The dosage of the formulation according to the present invention that is used in therapy will, of course, depend upon the nature of the pharmacologically active substance and the particular therapy.

According to further aspect of the present invention there is provided the use of a modified water-soluble polysaccharide as an excipient in a pharmaceutical formulation (preferably a modified release formulation), which modified water-soluble polysaccharide is obtainable by:
a) forming a precipitate of a water-soluble polysaccharide by contacting a solution of said polysaccharide with a solvent in which said polysaccharide is poorly soluble/insoluble; or
b) milling a water-soluble polysaccharide wherein the water-soluble polysaccharide is a hydroxyethyl cellulose.

The preferred modified water-soluble polysaccharides are those described above in relation to the modified release formulations. More preferably the modified polysaccharide is a modified HEC. Especially preferred modified water-soluble polyssacharides are those which when compressed into a tablet have a tablet hardness of more than 6kP, more preferably at least 7kP, for example from 6.5 to 40kP, more preferably from 7 to 35kP. Suitable conditions for determining the tablet hardness of the modified water-soluble polysaccharide are as hereinbefore described.

In a preferred embodiment of this aspect of the invention there is provided the use of a modified HEC as an excipient in a pharmaceutical formulation (preferably a modified release formulation), wherein said modified HEC has a tablet hardness of more than 6kP (preferably at least 7kP) when compressed into a tablet. The conditions for measuring the tablet hardness are as hereinbefore defined in relation to the first aspect of the invention.

### EXAMPLES

### Example 1: Modification of polysaccharides by poor solvent treatment.

A 1 % w/w water solution of the polysaccharide HEC G (Natirosol G from Hercules Ltd) or HEC HHX (Natrosol HHX from Hercules Ltd) was carefully poured into acetone while vigorously stirring. The final volume ratio acetone/water was 3/1. The precipitated polysaccharide was collected and washed twice with 500 mL acetone each time. During the washing the polysaccharide was carefully squeezed by hand to remove the water. The formed precipitate was torn into roughly 5 cm long threads that were dried in vacuum at 35°C typically 15 - 20 hours. The product was stored in a closed container at room temperature before use. Prior to use in the following examples, the precipitated polysaccharide was gently mortled to give a fine powder.

### Example 2: Modification of polysaccharides by milling.

Unmodified HEC G (Natrosol G from Hercules Ltd) or HEC HHX (Natrosol HHX from Hercules-Ltd) was milled either 5 min in 30 seconds intervals with an IKA A10 mill (knives) at 20000 rpm with water cooling, or with a microvibrator Retch MM mill (ball diameter = 2 cm) for 2x5 mm. The product was stored in a closed container at room temperature before use.

### Example 3: Granulation

Dry granulation was carried out by gently crushing tablets of directly compressed unmodified or modified HEC G or HEC HHX in a mortal and pestle. The granules obtained were then used after sieving (0.4-0.7 mm) for tabletting new formulations.

Wet granulation was carried out by mixing the unmodified or modified HEC G or HEC HHX with a granulation liquid selected from ethanol (95% or 99%); 10 %w/w PVP/99% ethanol; and 10%w/w HPC/99% ethanol to give a thick paste. The paste was then mortled gently to give granules of the unmodified or modified HEC G or HEC HHX. The granules were then dried overnight at 35 °C and sieved (0.4-0.7 mm).

### Example 4: Tabletting and hardness.

If not stated differently in the examples below the tablets were typically obtained by compressing 200 mg of unmodified or modified polysaccharide with a Killian excentric column press SP300 or with a Diaf press. Care was taken to use the same pressure during the compressions (approximately 20 kN). The tablet diameter was typically 8 mm, and convex standard punches were used in the machine. The hardness of the tablets was measured as the breaking force needed to crush the tablets with a Schleuniger-4M instruments. The drugs chosen to illustrate the use of the polysaccharides as modified release matrices were butyl paraben (p-hydroxybenzoic acid, SIGMA H-9503); the sodium salt of the HMG-CoA reductase inhibitor fluvastatin; the thrombin inhibitor described in Example 17 of WO 97/23499, Glycine, N-[1-cyclohexyl-2-[2-[[[[4-[(hydroxyimino)aminomethyl]-phenyl]methyl]amino]carbonyl]-1-azetidinyl]-2-oxoethyl]-, ethyl ester, [S-(R*,S*)]-) (hereinafter called Compound A); and the succinate and tartrate salts of the β-blocker metoprolol. These drugs are examples of drugs having solubilities ranging from very slightly soluble (butyl paraben; solubility = 0.2 mg/mL) to very soluble (fluvastatin; solubility> 50 mg/mL). The drugs were mixed together with the polysaccharides before tabletting as described in the following examples.

### Example 5: Release experiments.

The release experiments were carried out on tablets in a 0.1 M phosphate buffer pH =7 at a temperature of 37 °C, 50 rpm in a USP paddle system (Julabo). The tablets were placed in a cage-like basket, 1 cm above the paddle, with the tablet facing the flow direction. The concentrations were determined from measurements of the UV absorbance at appropriate wavelengths.

**Example 6:** The tablets shown in Table 1 were prepared from unmodified, or modified (by poor solvent treatment) HEC G polysaccharide obtained according to Example 1 by compressing the polysaccharide as described in Example 4 immediately after the modification of the polysaccharide.

**Table I. Tablet hardness, as measured by the force in kP needed to crush the tablets, for untreated and acetone-precipitated HEC G in accordance with Example 1. The tablets were stored for 0, 1 or 3 months in 40° C, 70% relative humidity.**

| | A | B | C | D | E | F |
|---|---|---|---|---|---|---|
| Tablet no 1 | 0.50 | 0.10 | 0.10 | 14.10 | 13.70 | 14.70 |
| " 2 | 0.40 | 0.10 | 0.10 | 14.40 | 11.30 | 13.40 |
| " 3 | 0.50 | 0.10 | 0.10 | 13.90 | 12.80 | 14.00 |

| | | | | | | |
|---|---|---|---|---|---|---|
| A, B C = non-treated stored 0, 1, and 3 months respectively D, E, F = acetone treated stored 0, 1, and 3 months respectively | | | | | | |

It is seen that substantial increase in tablet hardness was obtained after poor solvent modification of the polysaccharide.

**Example 7:** The tablets shown in Table II were prepared from unmodified, or modified HEC G polysaccharide obtained according to Example 1 and pressed according to Example 4 at different times after the modification of the polysaccharide.

**Table II. Tablet hardness, as measured by the force in kP needed to crush the tablets, for untreated and acetone-precipitated HEC G. The powders were stored before tabletting for 0, 1 or 3 months in 40° C, 70% relative humidity. The tablets were prepared 1-4 days before the measurements.**

| | A | B | C | D | E | F |
|---|---|---|---|---|---|---|
| Tablet no 1 | 0.50 | 0.10 | 0.10 | 14.10 | 4.90 | 3.10 |
| " 2 | 0.40 | 0.10 | 0.10 | 14.40 | 4.90 | 3.00 |
| " 3 | 0.50 | 0.10 | 0.10 | 13.90 | 4.60 | 3.70 |

| | | | | | | |
|---|---|---|---|---|---|---|
| A, B, C = non-treated stored 0,1, and 3 months respectively D, E, F = acetone treated stored 0, 1, and 3 months respectively | | | | | | |

It is seen that substantial increase in tablet hardness was obtained after poor solvent modification of the polysaccharide.

**Example 8:** The tablets shown in Table V were prepared from unmodified, or modified HEC HHX polysaccharide obtained according to Example 1, and pressed according to Example 4 immediately after the modification of the polysaccharide.

**Table V. Tablet hardness, as measured by the force in kP needed to crush the tablets, for untreated and acetone-precipitated HEC HHX. The tablets were stored for 0, 1 or 3 months in 40° C, 70% relative humidity.**

| | A | B | C | D | E | F |
|---|---|---|---|---|---|---|
| Tablet no 1 | 4.80 | 3.90 | 3.20 | 23.30 | 23.50 | 24.20 |
| " 2 | 5.40 | 4.00 | 3.10 | 23.80 | 23.10 | 22.00 |
| " 3 | 5.50 | 3.70 | 3.60 | 23.50 | 23.30 | 25.50 |

| | | | | | | |
|---|---|---|---|---|---|---|
| A, B, C = non-treated stored 0,1, and 3 months respectively D, E, F = acetone treated stored 0, 1, and 3 months respectively | | | | | | |

It is seen that substantial increase in tablet hardness was obtained after poor solvent modification of the polysaccharide.

**Example 9 :** The tablets shown in Table VI were prepared from unmodified, or modified HEC HHX polysaccharide obtained according to Example I, and pressed according to Example 4 at different times after the modification of the polysaccharide.

**Table VI Tablet hardness, as measured by the force in kP needed to crush the tablets, for untreated and acetone-precipitated HEC HHX. The powders were stored before tabletting for 0, 1 or 3 months in 40° C, 70% relative humidity. The tablets were prepared 1-4 days before the measurements.**

| | A | B | C | D | E | F |
|---|---|---|---|---|---|---|
| Tablet no 1 | 4.80 | 1.60 | 1.10 | 23.30 | 12.60 | 8.40 |
| " 2 | 5.40 | 1.80 | 1.00 | 23.80 | 12.40 | 8.30 |
| " 3 | 5.50 | 1.60 | 1.10 | 23.50 | 12.80 | 9.60 |

| | | | | | | |
|---|---|---|---|---|---|---|
| A, B, C = non-treated stored 0,1, and 3 months respectively D, E, F = acetone treated stored 0, 1, and 3 months respectively | | | | | | |

It is seen that substantial increase in tablet hardness was obtained after poor solvent modification of the polysaccharide.

**Example 10:** The influence by granulation of modified HEC G and HEC HHX was tested by experiments carried out according to example 3. Wet granulation of non-modified polysaccharides was however not possible due to the formation of unhandy polysaccharide mass of the polysaccharide and the granulation solvent Tablets were manufactured and the hardness measured according to example 4. For results see Table VII. It is seen that nearly comparable hardness was obtained after granulation when the polysaccharide either had been modified by poor solvent treatment or by milling (columns D,E,F, G). No changes could be detected when non-modified polysaccharide was dty granulated (column C). Substantial hardness persisted however when dry granulation was carried out on either poor solvent modified or milled polysaccharides (columns D, G). Also, wet granulation of poor solvent modified polysaccharide was possible (column H), although a decrease in hardness was obtained compared to not wet granulated samples (columns B, D). The results from bulk polysaccharide and non-granulated, poor solvent modified polysaccharide have been included for comparison (columns A, B). It should be emphasized that the results of columns A, C, and I were obtained with unmodified bulk polysaccharides as purchased.

**Table VII: Hardness of tablets (kP).**

| | A | B | C | D | E | F | G | H | I |
|---|---|---|---|---|---|---|---|---|---|
| HEC G | 0.5 | 14 | 0.8 | 14 | 5 | 13 | 7 | 7 | * |
| HEC HHX | 6 | 23 | 6 | 14 | 15 | 23 | 12 | 8 | * |

| | | | | | | | | | |
|---|---|---|---|---|---|---|---|---|---|
| * not possible to granulate A = bulk (supplied) polysaccharide; B = poor solvent treated polysaccharide; C = dry granulated bulk polysaccharide; D = dry granulated poor solvent modified polysaccharide; E = IKA A10 (knives) milled bulk polysaccharide; F =Retch MM (ball) milled bulk polysaccharide; G= dry granulated, Retch MM milled bulk polysaccharide H = wet granulated (PVP/ethanol) poor solvent modified polysaccharide I = wet granulated bulk polysaccharide. | | | | | | | | | |

**Example 11:** The influence from different milling times on the hardness of tablets produced were tested. Bulk polysaccharide samples were taken from the Retch MM mill as described in Example 2, but for the times shown in Table VIII. The samples were formed into tablets and the tablet hardness was measured as described in Example 4.

Table VIII shows that that after 10 minutes milling an approximate steady state value of the tablet hardness was attained.

**Table VIII: Milling time influence on tablet hardness. Average values of hardness (kP) are given at different times (min) after milling started.**

| Time/min | 0 | 1 | 2 | 3 | 4 | 5 | 10 | 15 | 20 |
|---|---|---|---|---|---|---|---|---|---|
| HEC G | 0.6 | 1.4 | 3.2 | 3.4 | 4 | 6 | 10 | 11 | 12 |
| HEC HHX | 6 | 8 | 10 | 11 | 12 | 16 | 20 | 24 | Nm |

| | | | | | | | | | |
|---|---|---|---|---|---|---|---|---|---|
| nm = not measured | | | | | | | | | |

**Example 12:** Release of butylparaben from HEC HHX tablets. The tablets were obtained as those of Example 8 but with butylparaben included. The release experiments were carried out according to Example 5.

**Table IX. Release of butyl paraben as a function of time from HEC HHX tablets, expressed as percent released of the total amount of butyl paraben. (The total amount butyl paraben was taken as the value obtained after complete dissolution of the tablets.) Each datapoint represents a mean of two separate experiments.**

| **A** | **B** | **A** | **C** | **A** | **D** | **A** | **E** | **A.** | **F** | **A** | **G** |
|---|---|---|---|---|---|---|---|---|---|---|---|
| 17 | 0.73 | 19 | 0.41 | 27 | 5.69 | 27 | 4.35 | 15 | 3.41 | 15 | 2.93 |
| 40 | 2.03 | 32 | 1.21 | 61 | 9.23 | 61 | 7.23 | 32 | 5.82 | 32 | 4.99 |
| 70 | 3.86 | 72 | 2.69 | 92 | 12.04 | 92 | 9.37 | 60 | 8.99 | 60 | 7.79 |
| 106 | 5.91 | 108 | 429 | 124 | 14.78 | 124 | 11.47 | 95 | 12.46 | 95 | 10.91 |
| 149 | 8.68 | 151 | 6.50 | 201 | 20.92 | 201 | 16.44 | 121 | 14.88 | 121 | 13.09 |
| 216 | 12.85 | 218 | 9.99 | 273 | 26.53 | 273 | 20.63 | 157 | 18.31 | 16.15 | 157 |
| 292 | 17.36 | 294 | 14.01 | 368 | 32.93 | 368 | 25.71 | 209 | 23.87 | 209 | 20.89 |
| 386 | 23.18 | 388 | 18.91 | 490 | 40.32 | 490 | 31.62 | 296 | 29 | 296 | 25.87 |
| 485 | 29.05 | 487 | 23.73 | 608 | 47.30 | 608 | 37.17 | 363 | 33.96 | 363 | 30.55 |
| 1444 | 75.73 | 1446 | 60.82 | 1404 | 81.57 | 1404 | 66.85 | 432 | 38.69 | 432 | 34.54 |
| 1571 | 80.74 | 1573 | G4.97 | 1539 | 84.80 | 1539 | 70.99 | 505 | 44.05 | 505 | 39.15 |
| 1680 | 84.10 | 1682 | 68.18 | 1659 | 87.59 | 1659 | 74.40 | 1418 | 82.82 | 1418 | 77.65 |
| 1855 | 88.24 | 1857 | 72 | 1802 | 90.32 | 1802 | 77.96 | 1671 | 88.36 | 1671 | 83.41 |
| 3012 | 98.65 | 3014 | 92.51 | 1907 | 93.05 | 1907 | 80.37 | 1945 | 93.23 | 1945 | 89.99 |
| 5929 | 99.69 | 5931 | 99.46 | 2872 | 98.13 | 2872 | 94.88 | 2954 | 100.21 | 2954 | 100.01 |
| 6120 | 100.78 | 6122 | 100.13 | 3030 | 98.93 | 3030 | 96.61 | 3447 | 101.08 | 3447 | 101.11 |
| 6240 | 100.07 | 6242 | 100 | 3273 | 98.87 | 3273 | 98.91 | 4440 | 98.58 | 4440 | 98.95 |
| | | | | 4500 | 100.12 | 4500 | 100.32 | | | | |
| | | | | 5000 | 99.88 | 5000 | 99.63 | | | | |

| | | | | | | | | | | | |
|---|---|---|---|---|---|---|---|---|---|---|---|
| Columns A: Time in minutes for the experiment to the right of each column A; Columns B, D, and F: % released after 0, 1, and 3 months storage of tablets from unmodified HEC HHX, respectively. Columns C, E, and G: % released after 0, 1, and 3 months storage of tablets from acetone treated HEC HHX, respectively. | | | | | | | | | | | |

It is seen that extended release formulations could be achieved with the stronger tablets based on the modified polysaccharide.

**Example 13:** Release of butylparaben from HEC HHX tablets. The tablets were obtained as those of Example 9 but with butyl paraben included. The release experiments were carried out according to Example 5.

**Table X Release of butyl paraben as a function of time from HEC HHX tablets, expressed as percent released of the total amount of butyl paraben. (The total amount butyl paraben was taken as the value obtained after complete dissolution of the tablets.) Each data point represents a mean of two separate experiments. The tablets were prepared 1-4 days before the experiments.**

| **A** | **B** | **A** | **C** | **A** | **D** | **A** | **E** | **A** | **F** | **A** | **G** |
|---|---|---|---|---|---|---|---|---|---|---|---|
| 17 | 0.73 | 19 | 0.41 | 15 | 3.73 | 15 | 3.41 | 15 | 3.32 | 15 | 3.29 |
| 40 | 2.03 | 42 | 1.21 | 32 | 5.95 | 32 | 5.49 | 32 | 5.75 | 32 | 5.49 |
| 70 | 3.86 | 72 | 2.69 | 61 | 9.33 | 61 | 8.36 | 67 | 9.76 | 67 | 9.44 |
| 106 | 5.91 | 108 | 4.29 | 90 | 12.45 | 90 | 11.10 | 94 | 12.33 | 94 | 12.03 |
| 149 | 8.68 | 151 | 6.50 | 158 | 18.18 | 158 | 16.30 | 141 | 18.05 | 141 | 16.55 |
| 216 | 12.85 | 218 | 9.99 | 241 | 24.61 | 241 | 21.92 | 191 | 21.52 | 191 | 20.63 |
| 292 | 17.36 | 294 | 14.01 | 350 | 32.28 | 350 | 28.87 | 273 | 27.29 | 273 | 26.32 |
| 386 | 23.18 | 388 | 18.91 | 493 | 41.45 | 493 | 36.92 | 360 | 33.44 | 360 | 32.33 |
| 485 | 29.05 | 487 | 23.73 | 1338 | 77.51 | 1338 | 73.46 | 444 | 39.07 | 444 | 37.50 |
| 1444 | 75.73 | 1446 | 60.82 | 1496 | 81.94 | 1496 | 77.45 | 521 | 43.87 | 521 | 41.90 |
| 1571 | 80.74 | 1573 | 64.97 | 1700 | 89.81 | 1700 | 82.09 | 1447 | 82.38 | 1447 | 82.48 |
| 1680 | 84.10 | 1682 | 68.18 | 1833 | 91.91 | 1833 | 83.88 | 1650 | 91.06 | 1650 | 89.39 |
| 1855 | 88.24 | 1857 | 72 | 2871 | 100.28 | 2871 | 96.50 | 1955 | 96.64 | 1955 | 94.54 |
| 3012 | 98.65 | 3014 | 92.51 | 3175 | 99.53 | 3175 | 99.31 | 2925 | 100.50 | 2925 | 100.98 |
| 5929 | 99.69 | 5931 | 99.46 | 3360 | 100.28 | 3360 | 99.99 | 3149 | 99.49 | 3149 | 99.16 |
| 6120 | 100.78 | 6122 | 100.13 | | | | | 3360 | 100.03 | 3360 | 99.86 |
| 6240 | 100.07 | 6242 | 100 | | | | | | | | |

| | | | | | | | | | | | |
|---|---|---|---|---|---|---|---|---|---|---|---|
| Columns A: Time in minutes for the experiment to the right of each column A; Columns B, D, and F: % released from tablets after 0, 1, and 3 months storage of powder from unmodified HEC HHX, respectively. Columns C, E, and G: % released from tablets after 0,1, and 3 months storage of powder from acetone treated HEC HHX, respectively. | | | | | | | | | | | |

It is seen that extended release formulations could be achieved with the stronger tablets based on the modified polysaccharide.

**Example 14:** Release of butylparaben from HEC G tablets. The tablets were those of Example 6 but with butyl paraben included. The release experiments were carried out according to Example 5.

**Table XI. Release ofbutyl paraben as a function of time from HEC G tablets, expressed as percent released of the total amount of butyl paraben. (The total amount butyl paraben was taken as the value obtained after complete dissolution of the tablets.) Each data point represents a mean of two separate experiments.**

| **A** | **B** | **A** | **C** | **A** | **D** | **A** | **E** | **A** | **F** | **A** | **G** |
|---|---|---|---|---|---|---|---|---|---|---|---|
| 13 | 1.84 | 15 | 1.15 | 10 | 5.32 | 10 | 3.59 | 15 | 7 | 15 | 3.90 |
| 26 | 2.57 | 28 | 1.86 | 27 | 8.70 | 27 | 6.50 | 32 | 11.02 | 32 | 7.20 |
| 45 | 4.26 | 47 | 3.50 | 61 | 15.19 | 61 | 12.28 | 60 | 16.82 | 60 | 12.49 |
| 72 | 7.46 | 74 | 6.19 | 92 | 26.73 | 92 | 17.09 | 95 | 24.30 | 95 | 19.09 |
| 125 | 14.38 | 127 | 12.70 | 124 | 26.96 | 124 | 22.72 | 121 | 30.12 | 121 | 24.22 |
| 166 | 20.90 | 168 | 19.93 | 201 | 42.98 | 201 | 37-55 | 157 | 39.11 | 157 | 31.71 |
| 239 | 33.72 | 241 | 33.18 | 273 | 56.98 | 273 | 51.60 | 209 | 53.86 | 209 | 44.05 |
| 303 | 46.60 | 305 | 46.01 | 368 | 75.32 | 368 | 69.03 | 296 | 69.70 | 296 | 58.90 |
| 385 | 62.46 | 387 | 61.98 | 490 | 92.84 | 490 | 87.30 | 363 | 80.70 | 363 | 71.39 |
| 484 | 78.85 | 486 | 80.18 | 608 | 98.09 | 608 | 97.56 | 432 | 89.36 | 432 | 81.95 |
| 563 | 89.65 | 565 | 92.13 | 1404 | 100.13 | 1404 | 100.07 | 505 | 96.28 | 505 | 91.79 |
| 1462 | 100 | 1464 | 99.94 | 1539 | 99.81 | 1539 | 99.87 | 1418 | 99.81 | 1418 | 99.53 |
| 1673 | 100 | 1675 | 100.06 | | | | | 1671 | 99.26 | 1671 | 99.19 |
| | | | | | | | | 1945 | 100.64 | 1945 | 100.08 |
| | | | | | | | | 2954 | 102.58 | 2954 | 100.60 |

| | | | | | | | | | | | |
|---|---|---|---|---|---|---|---|---|---|---|---|
| Columns A: Time in minutes for the experiment to the right of each column A; Columns B, D, and F: % released after 0, 1, and 3 months storage of tablets from unmodified HEC G, respectively. Columns C, E, and G: % released after 0, 1, and 3 months storage of tablets from acetone treated HEC G, respectively. | | | | | | | | | | | |

It is seen that extended release formulations could be achieved with the stronger tablets based on the modified polysaccharide.

**Example 15:** Release of butylparaben from HEC G tablets. The tablets were those of Example 7 but with butyl paraben included. The release experiments were carried out according to Example 5.

**Table XII. Release of butyl paraben as a function of time from HEC G tablets, expressed as percent released of the total amount of butyl paraben. (The total amount butyl paraben was taken as the value obtained after complete dissolution of the tablets.) Each data point represents a mean of two separate experiments. The tablets were prepared 1-4 days before the experiments.**

| **A** | **B** | **A** | **C** | **A** | **D** | **A** | **E** | **A** | **F** | **A** | **G** |
|---|---|---|---|---|---|---|---|---|---|---|---|
| 13 | 1.84 | 15 | 1.15 | 15 | 5.09 | 15 | 3.46 | 15 | 9.95 | 15 | 3.34 |
| 26 | 2.57 | 28 | 1.86 | 32 | 8.51 | 32 | 5.86 | 32 | 14.25 | 32 | 6.17 |
| 45 | 4.26 | 47 | 3.50 | 61 | 13.91 | 61 | 9.89 | 67 | 22.26 | 67 | 12.10 |
| 72 | 7.46 | 74 | 6.19 | 90 | 19.37 | 90 | 13.92 | 94 | 28.04 | 94 | 16.61 |
| 125 | 14.38 | 127 | 12.70 | 158 | 33.45 | 158 | 24.21 | 141 | 38.94 | 141 | 25.26 |
| 166 | 20.90 | 168 | 19.93 | 241 | 49.36 | 241 | 37.52 | 191 | 49.97 | 191 | 34.65 |
| 239 | 33.72 | 241 | 33.18 | 350 | 72.24 | 350 | 56.37 | 273 | 68.13 | 273 | 49.48 |
| 303 | 46.60 | 305 | 46.01 | 493 | 93 | 493 | 79.44 | 360 | 84.20 | 360 | 65.17 |
| 385 | 62.46 | 387 | 61.98 | 1338 | 100.43 | 1338 | 99.52 | 444 | 95.70 | 444 | 79.76 |
| 484 | 78.85 | 486 | 80.18 | 1496 | 99.75 | 1496 | 99.77 | 521 | 98.39 | 521 | 90.05 |
| 563 | 89.65 | 565 | 92.13 | 1700 | 99.82 | 1700 | 100.52 | 1447 | 99.93 | 1447 | 99.54 |
| 1462 | 100 | 1464 | 99.94 | | | | | 1650 | 100.20 | 1650 | 100.63 |
| 1673 | 100 | 1675 | 100.06 | | | | | | | 1955 | 99.02 |
| | | | | | | | | | | 2925 | 100.81 |

| | | | | | | | | | | | |
|---|---|---|---|---|---|---|---|---|---|---|---|
| Columns A: Time in minutes for the experiment to the right of each column A; Columns B, D, and F: % released from tablets after 0, 1, and 3 months storage of powder from unmodified HEC G, respectively. Columns C, E, and G: % released from tablets after 0, 1, and 3 months storage of powder from acetone treated HEC G, respectively. | | | | | | | | | | | |

It is seen that extended release formulations could be achieved with the stronger tablets based on the modified polysaccharide.

**Example 16:** Release of fluvastatin from HEC G tablets. The tablets were obtained from acetone treated HEC G as described in example 1 and were composed of 20 mg fluvastatin, 147 mg HEC G, and 1.7 mg magnesium stearate compressed as described in Example 4.

**Table XIII. Release of fluvastatin as a function of time from HEC G tablets, expressed as percent released of the total amount of fluvastatin. (The total amount of fluvastatin was taken as the value obtained after complete dissolution of the tablets.) Each datapoint represents a mean of at least two separate experiments.**

| **Time/hours** | **% released** |
|---|---|
| 0.5. | 5 |
| 1 | 8 |
| 2 | 16 |
| 4 | 35 |
| 6 | 56 |
| 8 | 72 |
| 10 | 80 |
| 12 | 82 |

It is seen that extended release formulations could be achieved with the tablets based on the modified polysacohride.

### Example 17:

Release of fluvastatin from HEC HHX tablets. The tablets were obtained from acetone treated HEC HHX as described in Example 1 and were composed of 10 mg fluvastatin, 58 mg HEC HHX, and 0.7 mg magnesium stearate compressed as described in Example 4.

**Table XlV. Release of fluvastatin as a function of time from HEC HHX tablets, expressed as percent released of the total amount of fluvastatin. (The total amount of fluvastatin was taken as the value obtained after complete dissolution of the tablets.) Each datapoint represents a mean of at least two separate experiments.**

| **Time/hours** | **% released** |
|---|---|
| 0.5 | 3 |
| 1 | 5 |
| 2 | 9 |
| 4 | 15 |
| 6 | 20 |
| 8 | 24 |
| 10 | 27 |
| 12 | 30 |

It is seen that extended release formulations could be achieved with the tablets based on the modified polysacchride.

### Example 18:

Release of metoprolol succinate from HEC HHX tablets. The tablets were obtained from milled HEC HHX (IKA A10) as described in example 2 and were composed of 25 mg metoprolol succinate, 253 mg HEC HHX, and 2.5 mg magnesium stearyl fumarate compressed as described in example 4.

**Table XV. Release of metoprolol succinate as a function of time from HEC HHX tablets, expressed as percent released of the total amount of metoprolol. (The total amount of metoprolol was taken as the value obtained after complete dissolution of the tablets.) Each datapoint represents a mean of at least two separate experiments.**

| **Time/hours** | **% released** |
|---|---|
| 0.5 | 5 |
| 1 | 10 |
| 2 | 19 |
| 4 | 32 |
| 6 | 42 |
| 8 | 51 |
| 10 | 57 |
| 12 | 63 |
| 24 | 87 |

It is seen that extended release formulations could be achieved with the tablets based on the modified polysacchride.

### Example 19

Release of metoprolol tartrate from HEC HHX tablets. The tablets were obtained from milled HEC HHX (IKA A10) as described in example 2 and were composed of 25 mg metoprolol tartrate, 253 mg HEC HHX, and 2.5 mg magnesium, stearyl fumarate compressed as described in example 4.

**Table XVI. Release of wetoprolol tartrate as a function of time from HEC HHX tablets, expressed as percent released of the total amount of metoprolol. (The total amount of metoprolol was taken as the value obtained after complete dissolution of the tablets.) Each datapoint represents a mean of at least two separate experiments.**

| **Time/hours** | **% released** |
|---|---|
| 1 | 18 |
| 2 | 29 |
| 4 | 44 |
| 8 | 65 |
| 12 | 77 |
| 16 | 89 |
| 20 | 91 |
| 24 | 93 |

It is seen that extended release formulations could be achieved with the tablets based on the modified polysacchride.

### Example 20:

Release of compound A from HEC G tablets. The tablets were obtained from milled HEC G (IKA A10) as described in example 2 and were composed of 25 mg compound A, 200 mg HEC G, and 2.5 mg magnesium stearyl fumarate compressed as described in example 4.

**Table XVII. Release of compound A as a function of time from HEC G tablets, expressed as percent released of the total amount of compound A. (The total amount of compound A was taken as the value obtained after complete dissolution of the tablets.) Each datapoint represents a mean of at least two separate experiments.**

| **Time/hours** | **% released** |
|---|---|
| 0.5 | 13 |
| 1 | 21 |
| 2 | 41 |
| 4 | 73 |
| 6 | 96 |
| 8 | 100 |
| 24 | 100 |

### Example 21:

Release of compound A from HEC HHX tablets. The tablets were obtained from milled HEC HHX (IKA A10) as described in example 2 and were composed of 25 mg compound A, 200 mg HEC HHX, and 2.5 mg magnesium stearyl fumarate compressed as described in example 4.

**Table XVIII. Release of compound A as a function of time from HEC HHX tablets, expressed as percent released of the total amount of compound A. (The total amount of compound A was taken as the value obtained after complete dissolution of the tablets.) Each datapoint represents a mean of at least two separate experiments.**

| **Time/hours** | **% released** |
|---|---|
| 0.5 | 4 |
| 1 | 5 |
| 2 | 8 |
| 4 | 13 |
| 6 | 19 |
| 8 | 25 |
| 24 | 78 |

**Example 22:** Bulk (supplied) polysacchride, IKA A10 (knives) milled, Retch MM (ball) milled, and poor solvent modified HEC G and HEC HHX were characterized by SEM pictures. It was found that significant changes of the polysacchride particles occurred after the different modification procedures, e g the bulk polysacchride particles were extended cylinders, the milled composed of more spherical particles, while the poor solvent modified showed a flake like appearance.

**Example 23:** Three batches of Natrosol HEC HHX were studied to reveal differences between modified, and non-modified polysaccharide:
A: bulk (supplied) polysaccharide;
B: ball milled according to example 2;
C: ball milled, compressed to a tablet according to example 4, and finally dry granulated according to example 3.

The powders were then analysed using Powder X-ray diffractometry and FT-Raman spectroscopy.

Powder X-ray diffractometry: Since only small differences between batch A and C could be detected, B was not used in this study. Both samples A and C had low crystallinity, showing a broad band around 22°2Θ and a few other weak features (around 8 and 43°2Θ). The spectra suggested some difference in the crystallinity of the samples.

FT-Raman spectroscopy: The Raman spectrum of samples B and C had a larger diffuse background than A. Thus a change in particle properties could be assured. Further, around 1405 cm⁻¹ a difference was found between A (peak at 1407 cm⁻¹), B (peak at 1404 cm⁻¹), and C (peak at 1405 cm⁻¹). Also, the three samples differed in the spectrum around 875 cm⁻¹ with A = 875 cm⁻¹, B = 874 cm⁻¹, and C = 872 cm⁻¹. The changes observed around 1405 cm⁻¹ and 875 cm⁻¹ may indicate slight changes in molecular structures resulting from the milling procedure. Both these frequencies are typical alkane frequencies, with 1405 cm⁻¹ typical for C-H bending (e g methyl), and 875 cm⁻¹ being typical for C-H bending or C-C stretch.

## Claims

1. A pharmaceutical modified release formulation comprising a pharmacologically active substance and a modified water-soluble polysaccharide, which modified water-soluble polysaccharide is obtainable by:
a) forming a precipitate of a water-soluble polysaccharide by contacting a solution of said polysaccharide with a solvent in which said polysaccharide is poorly soluble or insoluble;
or
b) milling a water-soluble polysaccharide,
wherein the water-soluble polysaccharide is a hydroxyethyl cellulose.

2. A modified release formulation according to claim 1 wherein the hydroxyethyl cellulose is obtainable by etherifying one or more reactive hydroxyl groups in cellulose with ethylene oxide.

3. A modified release formulation according to any one of the preceding claims wherein the hydroxyethyl cellulose has a degree of substitution from 0.5 to 3.

4. A modified release formulation according to any one of the preceding claims wherein the modified water-soluble polysaccharide has a tablet hardness of more than 6kP.

5. A modified release formulation according to any one of the preceding claims wherein the modified water-soluble polysaccharide is a modified water-soluble polysaccharide obtainable by forming a precipitate of a water-soluble polysaccharide by contacting a solution of said polysaccharide in a first solvent with a solvent in which said polysaccharide is poorly soluble or insoluble, wherein the first solvent is selected from the group consisting of water, formic acid, dimethyl sulfoxide and methyl formamide, or a mixture thereof.

6. A modified release formulation according to claim 5 wherein the solvent in which said water-soluble polysaccharide is poorly soluble or insoluble is an organic solvent selected from the group consisting of a ketone, an alcohol, an ester, an organic acid, an ether, a nitrile, an aromatic hydrocarbon and an aliphatic hydrocarbon, or a mixture thereof.

7. A modified release formulation according to claim 6 wherein the organic solvent is selected from the group consisting of acetone, ethanol, methanol, isopropyl alcohol, ethyl acetate, ethyl lactate, methyl salicylate, acetic acid toluene and hexane, or a mixture thereof.

8. A modified release formulation according to claim 5 wherein the first solvent is miscible in all proportions with the solvent in which the water-soluble polysaccharide is poorly soluble or insoluble.

9. A modified release formulation according to any one of claim 1 to claim 4 wherein the modified water-soluble polysaccharide is obtainable by milling a water-soluble polysaccharide in a milling device selected from the group consisting of a ball mill, a knife mill, an air-jet mill and a hammer mill.

10. A modified release formulation according to any one of claim 1 to claim 4 wherein the modified water-soluble polysaccharide is obtainable by milling a water-soluble polysaccharide in a knife mill, wherein said mill contains at least one knife blade rotating at 1 to 30000 rpm.

11. A modified release formulation according to claim 10 wherein the at least one knife blade rotates at approximately 20000 rpm.

12. A modified release formulation according to claim 9 wherein the water-soluble polysaccharide is milled for a time of from 0.25 to 600 minutes.

13. A modified release formulation according to claim 9 wherein the water-soluble polysaccharide is milled at a temperature that is lower than the glass transition temperature of the water-soluble polysaccharide.

14. A modified release formulation according to any one of the preceding claims wherein the pharmacologically active substance is fluvastatin or a pharmaceutically acceptable salt thereof.

15. A modified release formulation according to any one of claims 1 to 13 wherein the pharmacologically active substance is glycine, N-[1-cyclohexyl-2-[2-[[[[4-[(hydroxyimino)aminomethyl]-phenyl]methyl]amino]carbonyl]-1-azetidinyl]-2-oxoethyl]-, ethyl ester, [S-(R*,S*)]-.

16. A modified release formulation according to any one of claims 1 to 13 wherein the pharmacologically active substance is metoprolol or a pharmaceutically acceptable salt thereof.

17. A modified release formulation according to any one of claims 1 to 13 wherein the pharmacologically active substance is felodipine.

18. A modified release formulation according to any one of the preceding claims wherein the modified release formulation is in the form of granules comprising the modified water-soluble polysaccharide and pharmacologically active substance.

19. A modified release formulation according to any one of the preceding claims wherein modified release formulation comprises a compressed tablet adapted for oral administration.

20. A modified release formulation according to any one of the preceding claims wherein the modified water-soluble polysaccharide comprises at least 25% by weight of the modified release formulation.

21. A modified release formulation according to any one of the preceding claims wherein the modified water-soluble polysaccharide is the only polymeric excipient present in the formulation.

22. A compressed modified release tablet adapted for oral administration comprising a pharmacologically active substance and a modified water-soluble polysaccharide, wherein the modified water-soluble polysaccharide is obtainable by:
a) forming a precipitate of a water-soluble polysaccharide by contacting an aqueous solution of said polysaccharide with a water-miscible organic solvent in which said polysaccharide is poorly soluble/insoluble; or
b) milling for a period of at least 5 minutes a water-soluble polysaccharide in a knife mill containing at least one blade rotating at 10000 to 30000 rpm
wherein the water-soluble polysaccharide is a hydroxyethyl cellulose.

23. A modified release tablet formulation comprising a pharmacologically active substance and a modified water-soluble polysaccharide wherein the modified water-soluble polysaccharide is a modified hydroxyethyl cellulose according to claim 1, **characterised in that** the tablet has a hardness of more than 6kP.

24. A process for the preparation of a modified release formulation according to claim 1, which process comprises preparing a mixture of the modified water-soluble polysaccharide and the pharmacologically active substance and compressing the mixture.

25. A pharmaceutical modified release formulation according to any one of claims 1 to 23 for use in therapy.

26. The use of a modified water-soluble polysaccharide as an excipient in a pharmaceutical formulation, which modified water-soluble polysaccharide is obtainable by:
a) forming a precipitate of a water-soluble-polysaccharide by contacting a solution of said polysaccharide with a solvent in which said polysaccharide is poorly soluble/insoluble; or
b) milling a water-soluble polysaccharide
wherein the water-soluble polysaccharide is a hydroxyethyl cellulose.

## Patentansprüche

1. Pharmazeutische Formulierung mit modifizierter Freisetzung, enthaltend eine pharmakologisch wirksame Substanz und ein modifiziertes wasserlösliches Polysaccharid, wobei das modifizierte wasserlösliche Polysaccharid erhältlich ist, indem man:
a) einen Niederschlag eines wasserlöslichen Polysaccharids bildet, indem man eine Lösung des Polysaccharids mit einem Lösungsmittel, in dem das Polysaccharid schlecht löslich oder unlöslich ist, in Kontakt bringt;
oder
b) ein wasserlösliches Polysaccharid mahlt,
wobei es sich bei dem wasserlöslichen Polysaccharid um eine Hydroxyethylcellulose handelt.

2. Formulierung mit modifizierter Freisetzung nach Anspruch 1, wobei die Hydroxyethylcellulose durch Verethern einer oder mehrerer reaktiver Hydroxylgruppen in Cellulose mit Ethylenoxid erhältlich ist.

3. Formulierung mit modifizierter Freisetzung nach einem der vorhergehenden Ansprüche, wobei die Hydroxyethylcellulose einen Substitutionsgrad von 0,5 bis 3 aufweist.

4. Formulierung mit modifizierter Freisetzung nach einem der vorhergehenden Ansprüche, wobei das modifizierte wasserlösliche Polysaccharid eine Tablettenhärte von mehr als 6 kP aufweist.

5. Formulierung mit modifizierter Freisetzung nach einem der vorhergehenden Ansprüche, wobei es sich bei dem modifizierten wasserlöslichen Polysaccharid um ein modifiziertes wasserlösliches Polysaccharid handelt, das sich erhalten läßt, indem man einen Niederschlag eines wasserlöslichen Polysaccharids bildet, indem man eine Lösung des Polysaccharids in einem ersten Lösungsmittel mit einem Lösungsmittel, in dem das Polysaccharid schlecht löslich oder unlöslich ist, in Kontakt bringt, wobei das erste Lösungsmittel aus der aus Wasser, Ameisensäure, Dimethylformamid und Methylformamid oder einer Mischung davon bestehenden Gruppe ausgewählt ist.

6. Formulierung mit modifizierter Freisetzung nach Anspruch 5, wobei es sich bei dem Lösungsmittel, in dem das wasserlösliche Polysaccharid schlecht löslich oder unlöslich ist, um ein organisches Lösungsmittel ausgewählt aus der aus einem Keton, einem Alkohol, einem Ester, einer organischen Säure, einem Ether, einem Nitril, einem aromatischen Kohlenwasserstoff und einem aliphatischen Kohlenwasserstoff oder einer Mischung davon bestehenden Gruppe handelt.

7. Formulierung mit modifizierter Freisetzung nach Anspruch 6, wobei das organische Lösungsmittel aus der aus Aceton, Ethanol, Methanol, Isopropylalkohol, Essigsäureethylester, Milchsäureethylester, Salicylsäuremethylester, Essigsäure, Toluol und Hexan oder einer Mischung davon bestehenden Gruppe ausgewählt ist.

8. Formulierung mit modifizierter Freisetzung nach Anspruch 5, wobei das erste Lösungsmittel in allen Verhältnissen mit dem Lösungsmittel, in dem das wasserlösliche Polysaccharid schlecht löslich oder unlöslich ist, mischbar ist.

9. Formulierung mit modifizierter Freisetzung nach einem der Ansprüche 1 bis 4, wobei das modifizierte wasserlösliche Polysaccharid erhältlich ist, indem man ein wasserlösliches Polysaccharid in einem aus der aus einer Kugelmühle, einer Messermühle, einer Luftstrahlmühle und einer Hammermühle bestehenden Gruppe ausgewählten Mahlvorrichtung mahlt.

10. Formulierung mit modifizierter Freisetzung nach einem der Ansprüche 1 bis 4, wobei das modifizierte wasserlösliche Polysaccharid erhältlich ist, indem man ein wasserlösliches Polysaccharid in einer Messermühle mahlt, wobei die Mühle wenigstens eine Messerklinge enthält, die bei 1 bis 30.000 U/min rotiert.

11. Formulierung mit modifizierter Freisetzung nach Anspruch 10, wobei wenigstens eine Messerklinge bei ungefähr 20.000 U/min rotiert.

12. Formulierung mit modifizierter Freisetzung nach Anspruch 9, wobei das wasserlösliche Polysaccharid über eine Zeitspanne von 0,25 bis 600 min gemahlen wird.

13. Formulierung mit modifizierter Freisetzung nach Anspruch 9, wobei das wasserlösliche Polysaccharid bei einer Temperatur gemahlen wird, die unter der Glasübergangstemperatur des wasserlöslichen Polysaccharids liegt.

14. Formulierung mit modifizierter Freisetzung nach einem der vorhergehenden Ansprüche, wobei es sich bei der pharmakologisch wirksamen Substanz um Fluvastatin oder ein pharmazeutisch annehmbares Salz davon handelt.

15. Formulierung mit modifizierter Freisetzung nach einem der Ansprüche 1 bis 13, wobei es sich bei der pharmakologisch wirksamen Substanz um [S-(R*,S*)]-N-[1-Cyclohexyl-2-[2-[[[[4-[(hydroxyimino)aminomethyl]phenyl]methyl]amino]carbonyl]-1-azetidinyl]-2-oxoethyl]ethylester handelt.

16. Formulierung mit modifizierter Freisetzung nach einem der Ansprüche 1 bis 13, wobei es sich bei der pharmakologisch wirksamen Substanz um Metoprolol oder ein pharmazeutisch annehmbares Salz davon handelt.

17. Formulierung mit modifizierter Freisetzung nach einem der Ansprüche 1 bis 13, wobei es sich bei der pharmakologisch wirksamen Substanz um Felodipin handelt.

18. Formulierung mit modifizierter Freisetzung nach einem der vorhergehenden Ansprüche, wobei die Formulierung mit modifizierter Freisetzung in Form eines das modifizierte wasserlösliche Polysaccharid und die pharmakologisch wirksame Substanz enthaltenden Granulats vorliegt.

19. Formulierung mit modifizierter Freisetzung nach einem der vorhergehenden Ansprüche, wobei die Formulierung mit modifizierter Freisetzung eine für die orale Verabreichung geeignete komprimierte Tablette umfaßt.

20. Formulierung mit modifizierter Freisetzung nach einem der vorhergehenden Ansprüche, wobei das modifizierte wasserlösliche Polysaccharid wenigstens 25 Gew.-% der Formulierung mit modifizierter Freisetzung umfaßt.

21. Formulierung mit modifizierter Freisetzung nach einem der vorhergehenden Ansprüche, wobei es sich bei dem modifizierten wasserlöslichen Polysaccharid um den einzigen in der Formulierung vorhandenen polymeren Exzipienten handelt.

22. Für die orale Verabreichung geeignete komprimierte Tablette mit modifizierter Freisetzung, enthaltend eine pharmakologisch wirksame Substanz und ein modifiziertes wasserlösliches Polysaccharid, wobei das modifizierte wasserlösliche Polysaccharid erhältlich ist, indem man:
a) einen Niederschlag eines wasserlöslichen Polysaccharids bildet, indem man eine wäßrige Lösung des Polysaccharids mit einem wassermischbaren organischen Lösungsmittel, in dem das Polysaccharid schlecht löslich/unlöslich ist, in Kontakt bringt; oder
b) ein wasserlösliches Polysaccharid wenigstens 5 min lang in einer Messermühle, die eine bei 10.000 bis 30.000 U/min rotierende Klinge enthält, mahlt,
wobei es sich bei dem wasserlöslichen Polysaccharid um eine Hydroxyethylcellulose handelt.

23. Tablettenformulierung mit modifizierter Freisetzung, enthaltend eine pharmakologisch wirksame Substanz und ein modifiziertes wasserlösliches Polysaccharid, wobei es sich bei dem modifizierten wasserlöslichen Polysaccharid um eine Hydroxyethylcellulose nach Anspruch 1 handelt, **dadurch gekennzeichnet, daß** die Tablette eine Härte von mehr als 6 kP aufweist.

24. Verfahren zur Herstellung einer Formulierung mit modifizierter Freisetzung nach Anspruch 1, bei dem man eine Mischung des modifizierten wasserlöslichen Polysaccharids und der pharmakologisch wirksamen Substanz herstellt und die Mischung komprimiert.

25. Pharmazeutische Formulierung mit modifizierter Freisetzung nach einem der Ansprüche 1 bis 23 zur Verwendung in der Therapie.

26. Verwendung eines modifizierten wasserlöslichen Polysaccharids als Exzipient in einer pharmazeutischen Formulierung, wobei das modifizierte wasserlösliche Polysaccharid erhältlich ist, indem man:
a) einen Niederschlag eines wasserlöslichen Polysaccharids bildet, indem man eine Lösung des Polysaccharids mit einem Lösungsmittel, in dem das Polysaccharid schlecht löslich oder unlöslich ist, in Kontakt bringt;
oder
b) ein wasserlösliches Polysaccharid mahlt,
wobei es sich bei dem wasserlöslichen Polysaccharid um eine Hydroxyethylcellulose handelt.

## Revendications

1. Formulation pharmaceutique à libération modifiée comprenant une substance pharmacologiquement active et un polysaccharide modifié soluble dans l'eau, le polysaccharide modifié soluble dans l'eau pouvant être obtenu par :
a) formation d'un précipité d'un polysaccharide soluble dans l'eau par mise en contact d'une solution dudit polysaccharide avec un solvant dans lequel ledit polysaccharide est peu soluble ou insoluble; ou
b) broyage d'un polysaccharide soluble dans l'eau,
dans laquelle le polysaccharide soluble dans l'eau est une hydroxyéthylcellulose.

2. Formulation à libération modifiée selon la revendication 1 dans laquelle l'hydroxyéthylcellulose peut être obtenue par éthérification d'un ou de plusieurs groupes hydroxyle réactifs de la cellulose avec de l'oxyde d'éthylène.

3. Formulation à libération modifiée selon l'une quelconque des revendications précédentes dans laquelle l'hydroxyéthylcellulose a un degré de substitution de 0,5 à 3.

4. Formulation à libération modifiée selon l'une quelconque des revendications précédentes dans laquelle le polysaccharide modifié soluble dans l'eau a une dureté en comprimé de plus de 6 kP.

5. Formulation à libération modifiée selon l'une quelconque des revendications précédentes dans laquelle le polysaccharide modifié soluble dans l'eau est un polysaccharide modifié soluble dans l'eau pouvant être obtenu par formation d'un précipité d'un polysaccharide soluble dans l'eau par mise en contact d'une solution dudit polysaccharide dans un premier solvant avec un solvant dans lequel ledit polysaccharide est peu soluble ou insoluble, dans laquelle le premier solvant est choisi dans le groupe constitué par l'eau, l'acide formique, le diméthylsulfoxyde et le méthylformamide, ou un mélange de ceux-ci.

6. Formulation à libération modifiée selon la revendication 5 dans laquelle le solvant dans lequel ledit polysaccharide soluble dans l'eau est peu soluble ou insoluble est un solvant organique choisi dans le groupe constitué par une cétone, un alcool, un ester, un acide organique, un éther, un nitrite, un hydrocarbure aromatique et un hydrocarbure aliphatique, ou un mélange de ceux-ci.

7. Formulation à libération modifiée selon la revendication 6 dans laquelle le solvant organique est choisi dans le groupe constitué par l'acétone, l'éthanol, le méthanol, l'alcool isopropylique, l'acétate d'éthyle, le lactate d'éthyle, le salicylate de méthyle, l'acide acétique, le toluène et l'hexane, ou un mélange de ceux-ci.

8. Formulation à libération modifiée selon la revendication 5 dans laquelle le premier solvant est miscible en toutes proportions avec le solvant dans lequel le polysaccharide soluble dans l'eau est peu soluble ou insoluble.

9. Formulation à libération modifiée selon l'une quelconque des revendications 1 à 4 dans laquelle le polysaccharide modifié soluble dans l'eau peut être obtenu par broyage d'un polysaccharide soluble dans l'eau dans dispositif de broyage choisi dans le groupe constitué par un broyeur à billes, un broyeur à couteaux, un broyeur à jet d'air et un broyeur à marteaux.

10. Formulation à libération modifiée selon l'une quelconque des revendications 1 à 4 dans laquelle le polysaccharide modifié soluble dans l'eau peut être obtenu par broyage d'un polysaccharide soluble dans l'eau dans un broyeur à couteaux, ledit broyeur contenant au moins une lame de couteau tournant à 1 à 30 000 tours/min.

11. Formulation à libération modifiée selon la revendication 10 dans laquelle la ou les lames de couteau tournent à environ 20 000 tours/min.

12. Formulation à libération modifiée selon la revendication 9 dans laquelle le polysaccharide soluble dans l'eau est broyé pendant une durée de 0,25 à 600 minutes.

13. Formulation à libération modifiée selon la revendication 9 dans laquelle le polysaccharide soluble dans l'eau est broyé à une température qui est inférieure à la température de transition vitreuse du polysaccharide soluble dans l'eau.

14. Formulation à libération modifiée selon l'une quelconque des revendications précédentes dans laquelle la substance pharmacologiquement active est la fluvastatine ou un de ses sels pharmaceutiquement acceptables.

15. Formulation à libération modifiée selon l'une quelconque des revendications 1 à 13 dans laquelle la substance pharmacologiquement active est l'ester éthylique de [S-(R*,S*)]-N-[1-cyclohexyl-2-[2-[[[[4-[(hydroxyimino)aminométhyl]phényl]méthyl]amino]-carbonyl]-1-azétidinyl]-2-oxoéthyl]glycine.

16. Formulation à libération modifiée selon l'une quelconque des revendications 1 à 13 dans laquelle la substance pharmacologiquement active est le métoprolol ou un de ses sels pharmaceutiquement acceptables.

17. Formulation à libération modifiée selon l'une quelconque des revendications 1 à 13 dans laquelle la substance pharmacologiquement active est la félodipine.

18. Formulation à libération modifiée selon l'une quelconque des revendications précédentes dans laquelle la formulation à libération modifiée est sous forme de granulés comprenant le polysaccharide modifié soluble dans l'eau et une substance pharmacologiquement active.

19. Formulation à libération modifiée selon l'une quelconque des revendications précédentes dans laquelle la formulation à libération modifiée comprend un comprimé destiné à l'administration orale.

20. Formulation à libération modifiée selon l'une quelconque des revendications précédentes dans laquelle le polysaccharide modifié soluble dans l'eau comprend au moins 25% en poids de la formulation à libération modifiée.

21. Formulation à libération modifiée selon l'une quelconque des revendications précédentes dans laquelle le polysaccharide modifié soluble dans l'eau est le seul excipient polymère présent dans la formulation.

22. Comprimé à libération modifiée destiné à l'administration orale comprenant une substance pharmacologiquement active et un polysaccharide modifié soluble dans l'eau, dans lequel le polysaccharide modifié soluble dans l'eau peut être obtenu par :
a) formation d'un précipité d'un polysaccharide soluble dans l'eau par mise en contact d'une solution aqueuse dudit polysaccharide avec un solvant organique miscible avec l'eau dans lequel ledit polysaccharide est peu soluble/insoluble, ou
b) broyage pendant une durée d'au moins 5 minutes d'un polysaccharide soluble dans l'eau dans un broyeur à couteaux contenant au moins une lame tournant à 10 000 à 30 000 tours/min
dans lequel le polysaccharide soluble dans l'eau est une hydroxyéthylcellulose.

23. Formulation de comprimé à libération modifiée comprenant une substance pharmacologiquement active et un polysaccharide soluble dans l'eau modifié, le polysaccharide soluble dans l'eau modifié étant une hydroxyéthylcellulose modifiée selon la revendication 1, **caractérisée en ce que** le comprimé a une dureté de plus de 6 kP.

24. Procédé de préparation d'une formulation à libération modifiée selon la revendication 1, ce procédé comprenant la préparation d'un mélange du polysaccharide modifié soluble dans l'eau et de la substance pharmacologiquement active et la compression du mélange.

25. Formulation pharmaceutique à libération modifiée selon l'une quelconque des revendications 1 à 23 à utiliser en thérapeutique.

26. Utilisation d'un polysaccharide modifié soluble dans l'eau comme excipient dans une formulation pharmaceutique, le polysaccharide modifié soluble dans l'eau pouvant être obtenu par :
a) formation d'un précipité d'un polysaccharide soluble dans l'eau par mise en contact d'une solution dudit polysaccharide avec un solvant dans lequel ledit polysaccharide est peu soluble/insoluble; ou
b) broyage d'un polysaccharide soluble dans l'eau
dans laquelle le polysaccharide soluble dans l'eau est une hydroxyéthylcellulose.
